# EUROPEAN PATENT APPLICATION

(11) **EP 2 484 388 A1**
(43) Date of publication of application: **08.08.2012**
(21) Application number: 11000937.0
(22) Date of filing: 05.02.2011
(51) Int. Cl.: A61L 27/34, A61L 29/08, A61L 29/18, A61L 31/10, A61L 31/14

(54) **Implantable or insertable MRI-detectable medical device having a coating comprising paramagnetic ions and a process for preparing it**

(71) Applicant: MaRVis Technologies GmbH, 52068 Aachen (DE)
(72) Inventor: Christian Meyer, 67059 Ludwigshafen am Rhein (DE); Björn Degel, 67454 Hassloch (DE); Klaus Düring, 50226 Frechen (DE)
(74) Representative: Schüssler, Andrea

(57) **Abstract**

The present invention concerns a medical device which can be detected by means of magnetic resonance imaging (MRI). The medical device is characterized by having mechanically stably attached a coating comprising paramagnetic ions which are directly and strongly encompassed by the coating polymers. The medical device comprises a modified envelope polymer providing chemically active free functional groups and a coating covalently bonded to the free functional groups of the envelope polymer at its surface. The coating contains statistically encompassed paramagnetic ions to render it MR visible.

## Description

The present invention relates to a medical device and a process for preparing it. In particular, the present invention concerns a medical device which can be detected by means of magnetic resonance imaging (MRI).

A detailed explanation of MRI can be found in the Internet at http:// en.wikipedia.org/wiki/Magnetic Resonance_Imaging.

Medical devices equipped with paramagnetic metallic compounds and/or a paramagnetic metal so that they are visible in MRI are known from EP 1 206 945 A1, WO 99/060920 A2, WO 2003/045462 A, and WO 2003/094975 A.

WO 87/02893 discloses poly-chelating substances for imaging enhancement and spectral enhancement for MRI. These substances comprise different complexes in which metal ions, in particular gadolinium ions, are immobilized.

The relaxivity of gadolinium(III) complexes is explained in chapter 1.6.1 of the Ph.D thesis (Inaugural Dissertation) by Daniel Storch, entitled "Neue, radioaktiv markierte Magnet-Resonanz-aktive Somatostatinanaloga zur besseren Diagnose und zielgerichteten Radionuklidtherapie von neuroendokrinen Tumoren", Basel, 2005. The paramagnetic relaxation of the water molecules which are present in the vicinity of the gadolinium(III) ion is the result of the dipole-dipole-interaction between the nuclear spin and the fluctuating local magnetic field of the MRI scanner, caused by the unpaired electrons. The magnetic field around the paramagnetic center, i.e. the gadolinium(III) ion, decreases with increasing distance. Therefore, it is essential to locate the protons in close proximity to the metal ion. For gadolinium(III) complexes this means that the water molecules are to be transported into the first coordination sphere of the metal ion. These "inner-sphere" H₂O molecules are exchanged with the surrounding water molecules and in this way transmit the paramagnetic effect.

DE 100 40 381 C1 discloses fluoroalkyl-containing complexes with residual sugars. These complexes can be provided with paramagnetic metal ions so that they can serve as contrast agents in magnetic resonance imaging. These metal ions are in particular the bivalent and trivalent ions of the elements of the atomic numbers 21 to 29, 42, 44 and 58 to 70. Suitable ions are, for instance, the chromium(III), iron(II), cobalt(II), nickel(II), copper(II), praseodymium(III), neodymium(III), samarium(III) and ytterbium(III) ions. Gadolinium(III), erbium(III), dysprosium(II-I), holmium(III), erbium(III), iron(III) and manganese(II) ions are particularly preferred because of their strong magnetic moment.

WO 99/060920 A, WO 2002/022186 A, WO 2003/094975 A and EP 1 501 552 A each show a coating for a medical device comprising a paramagnetic ion which is bound in a chelate complex which itself is covalently coupled to the coating polymer. In particular, the paramagnetic ion is gadolinium. This coating is visible in MRI. Adaptation of the doping of the coating with the gadolinium-chelate complex concurrent with the necessary control of coating thickness and water uptake is difficult to handle. Further, the coating is not stably attached to the surface of the polymer and sensitive to mechanical abrasion. This may lead to the release of coating polymer particles containing gadolinium-chelate complex and result in free flowing of the particles in the bloodstream.

WO 1998/049206, WO 2001/039814, WO 2007/080387, WO 2008/029082 and WO 2009/019477, all of the applicant Polybiomed Ltd., concern different methods how to provide a polymeric surface with hydrophilic properties.

One object of the present invention is to further improve medical devices comprising paramagnetic ions. These improved medical devices are suitable to be inserted into a human or animal body and are very versatile in their use in MRI examinations.

This object is achieved by a medical device comprising the features of claim 1 and 10. Advantageous embodiments are indicated in the sub-claims.

Throughout the specification the term "medical device" is used in a broad sense to refer to any medicinal device, tool, instrument or other object. The medical devices of the present invention are particularly useful as any type of guidewires, catheters (including vascular and non-vascular, esophageal, peritoneal, peridural, nephrostomy catheters), grafts, biopsy needles, puncture needles, cannulae, intralumenal medical devices, endotracheal tubes, and ablation devices. They can be introduced or implanted in a "target" or "target object". The target or target object is all or a part of the human or animal body. The medical device of the present invention particularly may be brought into cavities of the target (object). These cavities are particularly blood vessels, neuronal ways, any organs (whole or part) or tissues (whole or part).

The medical device of the present invention is characterized by having mechanically stably attached a coating comprising paramagnetic ions which are directly and strongly encompassed by the coating polymers. The medical device of the present invention comprises a modified envelope polymer providing chemically active free functional groups and a coating covalently bonded to the free functional groups of the envelope polymer at its surface. The coating contains statistically encompassed paramagnetic ions to render it MR visible.

The basis of the medical device of the present invention may be any MR-safe medical device covered by an envelope polymer, which does not lead to electric conductivity and/or heating and is not dislocated when present in the magnetic and radiofrequency fields during MRI.

Mechanically stable attachment of the coating to the surface of the medical device is achieved by employing a modified surface/envelope polymer within the medical device which provides chemically active free functional groups at the surface. The coating is de novo synthesized by the incubation of the medical device having the active free functional groups at its surface in a solution of one or more coating polymers. By the reaction of the functional groups of the one or more coating polymers with the active free functional groups of the surface/envelope polymer of the medical device the polymer coating is covalently bonded to the surface/envelope polymer. Covalent bonding is the mechanically most stable means of attachment of a coating to a polymer surface.

Direct encompassing of paramagnetic ions by the coating polymer molecules is achieved by constructing a network of polymer layers (at least one, preferably two or more) which contribute free functional groups for encompassing of the paramagnetic metal ion. In contrast to a chelate complex, which is highly symmetric and reproducible, the network for binding of paramagnetic ions according to the present invention is created in a statistical manner, i.e. by formation of a huge variety of different non-symmetric arrangements of functional groups and conformations of these groups in the binding pockets. The binding pockets are "mini-cavities" within the coating which are capable to encompass and surround the paramagnetic metal ion. They contribute highest possible stability for encompassing of the paramagnetic ions whereas the binding strength of the paramagnetic ion statistically varies from binding pocket to binding pocket. By the variation of the chemistry of the coating a huge variety of concentrations of the paramagnetic ion, strength of the binding, thickness of the coating and water uptake capacity of the coating can be realized. The washing stringency for the coating loaded with the paramagnetic ion may be appropriately chosen to obtain a coating with a preset minimum binding stability of the encompassed paramagnetic ions in order to ensure highest possible patient safety, i.e. minimal release of paramagnetic ions.

In a specific embodiment the medical device may comprise at least one rod shaped body (in the following: rod). The rod is preferably a rod as described in WO 2007/000148 A2, WO 2009/141165 A2 or EP application 10 187 863. Therefore, full reference is made to the disclosure of these documents and those documents are incorporated here by reference.

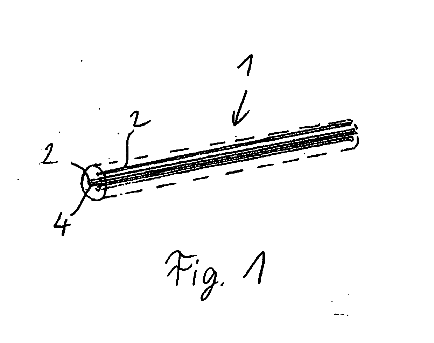

As shown in Fig. 1 the rod 1 comprises one or more non-metallic filaments 2 and a non-ferromagnetic matrix material 3 (in the following: matrix). The matrix material encloses and/or agglutinates the filaments and the matrix material is preferably a regular or high temperature resistant epoxy resin, PVC or synthetic rubber.

For some particular uses the rods or alternatively the envelope polymer may be doped with marker particles for generating a signal in an X-ray or MRI process. These particles (e.g. iron oxide or iron) are embedded in the matrix material or the envelope polymer. Different marker particles may be used, whereas in a medical device differently doped and/or undoped rods or envelope polymers can be incorporated. Simply by use of different markers various medical devices having different characteristics in X-ray or MRI processes can be easily and cost-efficiently manufactured in the same process.

The filaments provide a high strength to the rods in longitudinal direction. Medical devices comprising these rods frequently are designed for being introduced into a blood vessel, an organ (e.g. heart, liver, kidney or lung) or the brain. Therefore, a strong force can be applied to these medical devices in longitudinal direction during introduction of these devices into the body cavity or when pulling them out thereof. This force is taken up by the rods. On the other hand the medical devices have to provide a certain flexibility to guide them along curves of the body cavity.

The filaments are usually made of glass fibers. It is also possible that the filaments are ceramic fibers or polyamide or aramid (e.g. Kevlar^{®} fibers as long as the fibers provide the necessary strength in longitudinal and lateral direction. It is possible to also use other kinds of fibers as long as the fibers do not provide magnetic and electrically conductive properties.

The rods used for the medical device of the present invention are preferably produced by means of a micro-pultrusion process. In such a micro-pultrusion process a roving (= a group of several filaments being arranged in parallel to each other) is pultruded together with the matrix material in which marker particles, if applicable, can be contained. It is preferred that the number of filaments is at least four or even more, e.g. at least six or at least ten. The amount of filaments has a strong influence on the mechanical properties of the rods. In an alternative embodiment yarns can be used instead of rovings for the production of the rods. In such yarns the filaments are drilled or braided. However, rovings are preferred, as the drilled or braided structure of the yarns may cause a corresponding structure at the surface of the produced rods. Rods having a smooth surface instead of such a structured surface are preferred because it is easier to use them in a subsequent extrusion process.

According to a further aspect of the present invention a medical device comprises one or more rod shaped bodies, each comprising
- one or more non-metallic filaments and
- a non-ferromagnetic matrix material,
wherein the matrix material encloses and/or agglutinates the filaments and marker particles for generating a signal in an X-ray or magnetic resonance imaging process,
and an envelope polymer in which the one or more rod shaped bodies are embedded, wherein a cord is embedded either in the matrix material or in the envelope polymer, wherein the cord is more flexible than the non-metallic filaments.

The cord preferably is a thin cord having a high tensile strength and consists of a material with a higher flexibility than the filaments. Suitable cords are e.g. polyamide filaments, aramid filaments, polyethylene terephthalate (PET) filaments, rayon filaments (e.g. HL fiber), cotton filaments, or hemp filaments. The cord extends along the total device or rod, resp., and is directed in the longitudinal direction of the device or rod, resp. Such a cord does not break if it is bent. This means that if the rod or a medical device incorporating such a rod breaks, the broken parts are still connected by means of the cord. Thereby, it is ensured that even if such a breakage occurs in the human or animal body during the medical intervention the broken parts can be safely pulled out. If the cord is positioned in a rod, it is advantageously arranged in the center of the rod.

In a preferred embodiment the medical device according to the present invention comprises as the envelope polymer a biocompatible material. Such biocompatible materials are available on the market e.g. under the trade names Mediprene^{®} or Tecoflex^{™}. Tecoflex^{™} is an elastic polymer material which is based on polyurethane (PU). Mediprene^{®} is a thermoplastic elastomer made from SEBS (styrene-ethylenebutylene-styrene-elastomer) which is primarily used for medical purposes. Mediprene may be purchased from Elasto AB, Sweden.

The flexible and elastic envelope polymer provides a certain shape to the medical device. If the medical device is one which contains rods, the envelope polymer encloses the rods. Hence, the medical devices consist of a multi-composite material comprising the rods as a kind of reinforcing material and the envelope polymer as embedding and agglutinating material. The mechanical properties of a medical device are mainly defined by the rods.

According to the present invention an outer coating is covalently coupled to the surface of the medical device. This outer coating shall provide a smooth, preferably lubricious, outer surface to the medical device and in a preferred embodiment provide paramagnetic properties. Hence, the envelope polymer is modified by compounding, i.e. mechanically mixing it, with solid or liquid chemical compounds having one or more functional groups, preferably amino and/or carboxylic groups. These chemical compounds are preferably mono-, di- or polycarboxylic acids, mono-, di- or polyamines, polyethyleneimine, or polyallylamine. The surface functional groups, preferably the carboxyl groups/amino groups, are reacted with corresponding functional groups of a coating polymer, preferably with amino groups/carboxyl groups, to obtain a covalent bond, preferably an amide bond. These reactions are according to known peptide chemistry processes and well known to a person skilled in the art. The residual functional groups (e.g. the remaining carboxyl/amine groups) are then at least partially chemically crosslinked by a crosslinker.

To provide the medical device with paramagnetic properties the medical device is impregnated with an aqueous solution of a paramagnetic marker.

A critical parameter in constructing a valuable and medically useful coating is the exchange rate of the water molecules associated with the paramagnetic ions. If the exchange rate is to high the MRI signal may not be recordable at all or may provide a signal at a different location than that of the paramagnetic ion itself as the magnetically enhanced water molecule may have moved away a significant distance during the time period between application of the MR (RF) pulse and measuring of the echo ("echo time"). The flexibility in the design and optimization of the present coating allows directed balancing of water uptake and water exchange rates in order to obtain a good MR image without compromising quality of visualization of the body tissue.

In a particularly preferred embodiment the coating according to the present invention may contain a further layer of a lubricious polymer which optionally is crosslinked. This lubricious polymer may be the same compound as the coating polymer or a different polymer or a conventional hydrogel.

In another embodiment of the present invention the coating is first covalently attached to small polymer particles, e.g. micro- or nano-particles, preferably polystyrene nano-particles. The coated polymer particles are then compounded with the surface/envelope polymer of the medical device which is used to manufacture the medical device. Optionally a lubricious coating has to be attached to the surface of the medical device. For that purpose the chemical compounds which provide chemically active free functional groups as described above are co-compounded along with the coated polymer particles with the surface/envelope polymer and a lubricious coating is covalently bonded to the functional groups of the surface/envelope polymer. Alternatively, the coating of the particles itself may provide a sufficient number of free functional groups to which the lubricious coating may be covalently coupled.

Examples of "mono-, di- or polycarboxylic acids" are oxalic acid, malonic acid, succinic acid, glutaric acid, adipinic acid, maleinic acid, (poly)acrylic acid, (poly)methacrylic acid, (poly)maleic acid, (poly)aspartic acid, (poly)glutamic acid, alginic acid or pectinic acid. Their linear copolymers, crosslinked copolymers, graft copolymers and block copolymers can be also used and are included within the scope of the invention. Particularly preferred is an acrylic acid polymer or an acrolein-acrylic acid copolymer like POC AS 5060 (Evonik Industries, Essen, Germany).

Examples of "mono-, di- or polyamines" are amino acids (e.g. glycine, lysine, glutamine etc.), ethylenediamine, trimethylenediaminepolyvinylamine, polylysine, 2,4-diaminopropane, 1,3-diaminobutane. Particularly preferred is polyvinylamine.

Examples of "lubricious polymers" or "(outer) coating polymers" are poly(L-lysine), polyvinylamine, proteins, collagen, cellulosic polymers, (modified) dextran, gelatin, (carboxymethyl) starch, hyaluronic acid, chitin, polyvinylalcohol, polyvinylpyrrolidone (PVP) or polyvinylpolypyrrolidone (PVPP). The lubricious polymer shall provide a lubricious surface to the medical device.

The term "impregnating" means any process for application of an aqueous salt solution to a surface, e.g. dipping, spraying, brushing, soaking etc. In a preferred embodiment the impregnation period is between 20 and 60 minutes, preferably about 30 minutes.

"Paramagnetic marker" means any chemical compound comprising paramagnetic ions selected from the group of praesodynium (III), neodymium (III), samarium (III), ytterbium (III), gadolinium (III), terbium (III), dysprosium (III), holmium (III) and erbium (III), with gadolinium (III), dysprosium (III) and ytterbium (III) being preferred. Gadolinium, dysprosium and similar metals are passive positive markers as they reduce the proton spin relaxation time of associated water molecules. Due to their specific characteristics and influences on the magnetic properties (relaxation times) of the protons in the water molecules located directly adjacent to the rods or medical devices these MRI markers can be detected by common water-proton adjusted MRI sequences.

Selection of the crosslinker is not restricted by any limitations. It may be a mono-, bi-or polyfunctional compound. Examples of crosslinking reagents are bifunctional epoxides, isocyanates, chlorotriazines, amidines or aldehydes. In a preferred embodiment of the invention the crosslinking agent is an alcoholic solution of ethylene glycol diglycidyl ether. The amount/concentration of the crosslinker is between 3 and 25%, preferably 10-20%, of the reactive groups.

Particularly preferred is to use Mediprene^{®} as the envelope polymer and to compound it, i.e. to mix it mechanically, with an acrolein-acrylic acid-copolymer (e.g. POC AS 5060, Evonik Industries, Essen, Germany), to obtain a content of 5, 10, 20, 30 or 40 or higher than 40% (w/w) POC within the Mediprene®. All amounts therebetween are also suitable and may be used. Particularly preferred are amounts between 10 and 20%. In a specific embodiment this modified envelope polymer is then used in an extrusion process to embed and agglutinate the above described rods and/or cords to provide sufficient mechanical strength. After the extrusion the surface is activated and free surface carboxylic groups are reacted preferably with polyvinylamine or other polyamino polymers resulting in a covalent amide linkage. Activation of the medical device surface is made with a known activation reagent, e.g. HBTU, HATU, BOP, PyBOP. Free residual carboxyli/amino groups are then crosslinked to provide a stably attached coating at the surface of the medical device. Further carboxylic groups may be introduced into the polymeric layers of the medical device coating by impregnating the device with a 0.5-5% (preferably 1 %) solution of succinic acid anhydride in a suitable organic solvent (e.g. in dimethylformamide). If desired, one or more additional layers of a polyamine (e.g. polyvinylamine) may be brought onto the surface and at least partially crosslinked. These modified surfaces are suitable to incorporate paramagnetic markers by impregnating the medical device with an aqueous solution of a paramagnetic marker, preferably with a 0.5 mg/ml GdCl₃ solution. If desired, a further layer of a polyamine as a lubricous polymer (preferably polyvinylamine) may be brought onto the surface.

In a particularly preferred embodiment the envelope polymer (e.g. Mediprene) is modified by "spiking" with POC to provide free carboxylic groups. As mentioned above the prefered compositions are 5 - 20% Mediprene-POC, most preferably 10% POC. Then polyvinylamine (PVA) is covalently coupled via amide bonds to the carboxylic groups wherein the PVA layer may be provided in a multilayer. Then the PVA layer is slightly crosslinked. Thereafter one or more PVA layers are physically applied and may also be crosslinked. This leads to covalent linkages between the individual layers. The following treatment with succinic acid (butanedioic acid) anhydride provides free carboxylic groups in the PVA coating. This coating may then be impregnated with a paramagnetic marker (e.g. gadolinium chloride) solution which binds by the coordination of its free electron pairs to the carboxylic and/or amino groups of the coating. Thereafter, a further PVA layer may be applied and may be crosslinked. This step may be repeated several times until a thickness of the coating of preferably about 0,05 bis 0,10 mm is achieved.

Further optimization with regard to the binding stability of gadolinium may be achieved by (a) using different crosslinkers, (b) variation of the length of the crosslinkers, (c) variation of the concentration of the crosslinkers.

With respect to the coating containing passive MRI markers the goal is to have a) a strong signal and b) a confined and sharp signal. However, the stronger the signal is, the bigger are the artifacts which reduce the image sharpness. Preferably, the signal should be reasonably balanced in longitudinal (strong enough) and orthogonal direction (not to broad).

The invention is further described with respect to the Figures which show:
- Fig. 1:: Rod of a medical device
- Fig. 2:: 3 different test samples resulting in a strong gadolinium-derived MRI signal
The invention is further described in the Example:

### Example 1:

A 30 cm guidewire (MaRVis Technologies GmbH, Aachen, Germany) comprising Mediprene^{®} modified with an acrolein-acrylic acid-copolymer (POC AS 5060; 10 %) as the envelope polymer was coated with polyvinylamine. The coating was made according to known peptide chemistry processes by reacting polyvinylamine (15 % w/v in dimethylformamide) with the HBTU-activated surface of the guidewire. Then the chemical groups at the surface of the guidewire were crosslinked with a 0.7% (v/v) solution of ethylene glycol diglycidyl ether in isopropanol. The guidewire was then dipped into a 1.0% solution of succinic acid anhydride in dimethylformamide.

Subsequently the guidewire was dipped into a 0.25 % (w/v) aqueous gadolinium chloride solution and left in the solution until about 4.1 µg gadolinium salt was adsorbed by the coating of the guidewire. The decrease of the gadolinium concentration in the aqueous solution was determined according to standard methods. Subsequently a further layer of polyvinylamine was applied as described above. The guidewire was dried in a drying chamber at 80°C.

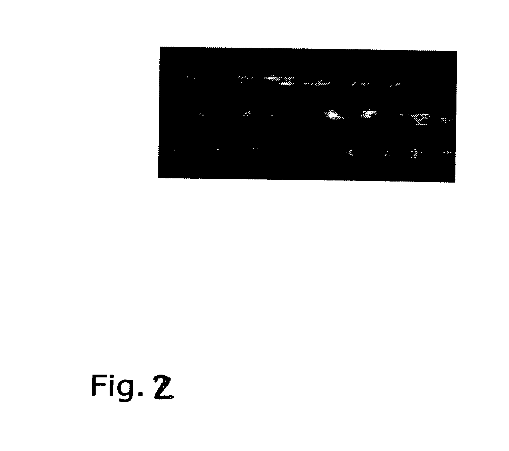

Coated and gadolinium-loaded test samples were analyzed in an MRI process (c.f. Fig. 2). In these tests the samples were placed in a water bath (water phantom) so that they were completely surrounded and covered by water. This water phantom was placed into the magnetic field of an MR scanner. There are standard measuring conditions ("MR sequences") in MRI systems for detection of the position and properties of the water-protons in the local magnetic field. The samples were tested with the "CA-angio" standard sequence employed on a Siemens Magnetom Symphony 1.5 Tesla MR scanner:

### CA-angio sequence

GRE/FLASH 3D, TR/TE = 4.3/1.38 ms, slice thickness: 0.5 mm,
FOV = 400×325 mm², matrix: 512×208, averages: 2, phase FOV: 81.25 %, percent sampling: 50 %, bandwidth: 515 Hz/px, flip angle: 16°, TA = 67 s, total number of slices: 88, phase encoding steps: 166 (208), slab thickness: 44 mm

## Claims

1. A medical device detectable by magnetic resonance imaging (MRI), said device comprising an envelope polymer that is modified by compounding it with at least one chemical compound having one or more chemically active free functional groups to provide a surface coating covalently bonded to the free functional groups of the modified envelope polymer, wherein paramagnetic ions are encompassed by the coating.

2. The medical device of claim 1, wherein the chemical compound is a solid or liquid mono-, di- or polycarboxylic acid, mono-, di- or polyamine, polyethyleneimine or polyallylamine.

3. The medical device of claim 1 or 2, wherein the surface coating is obtained by reacting the free functional groups with corresponding functional groups of the coating polymer to obtain a covalent bond.

4. The medical device of any of claims 1 to 3, wherein the covalent bond is an amide bond.

5. The medical device of any of claims 1 to 4, wherein the envelope polymer is a thermoplastic elastomer made from styrene-ethylene-butylene-styrene-elastomer.

6. The medical device of any of claims 1 to 5, wherein the chemical compound is a polyacrylic acid polymer or an acrolein-acrylic acid copolymer.

7. The medical device of claim 6, wherein the covalently bonded surface coating is obtained by reacting the polyacrylic acid polymer or acrolein-acrylic acid copolymer with polyvinylamine.

8. The medical device of any of claims 1 to 7, wherein the paramagnetic ions are selected from the group consisting of gadolinium (III), dysprosium (III), praesodynium (III), neodymium (III), samarium (III), ytterbium (III), terbium (III), holmium (III) and erbium (III).

9. The medical device of any of claims 1 to 8, wherein it is a guidewire, a catheter, a graft, a biopsy needle, a puncture needle, a cannula, an intralumenal medical device, an endotracheal tube, or an ablation device.

10. A process for preparing the medical device of any of claims 1 to 9, wherein the envelope polymer of a medical device is modified by compounding it with at least one chemical compound having one or more chemically active free functional groups to provide a surface coating covalently bonded to the free functional groups of the modified envelope polymer, wherein the coating is impregnated with paramagnetic ions.

11. The process of claim 10, wherein the free functional groups of the modified envelope polymer are carboxyl groups which react with amino groups of the coating polymer to obtain a covalent amide bond and residual amino groups are then at least partially chemically crosslinked by a crosslinker to provide the surface coating.

12. The process of claim 10 or 11, wherein the envelope polymer is a thermoplastic elastomer made from styrene-ethylene-butylene-styrene-elastomer which is chemically modified by mixing it with a polyacrylic acid polymer or an acrolein-acrylic acid copolymer.

13. The process of claim 11, wherein the amino groups are derived from polyvinylamine.

14. The process of any of claims 10 to 12, wherein further carboxyl groups are brought onto the surface of the medical device by impregnating the device with a solution of succinic acid anhydride in a organic solvent.

15. The process of claim 10, wherein the paramagnetic ions are a gadolinium (III), dysprosium (III), praesodynium (III), neodymium (III), samarium (III), ytterbium (III), terbium (III), holmium (III) or erbium (III) salt solution.
